# EUROPÄISCHE PATENTSCHRIFT

(11) **EP 1 711 453 B1**
(45) Veröffentlichungstag und Bekanntmachung des Hinweises auf die Patenterteilung: **01.12.2010**
(21) Anmeldenummer: 05700997.9
(22) Anmeldetag: 18.01.2005
(51) Int. Cl.: C07C 69/54, C07C 67/08, C07C 67/26

(54) **POLYMERDISPERSIONEN ODER -LÖSUNGEN MIT 3,4 DIHYDROXYPHENYLGRUPPEN**
POLYMER DISPERSIONS OR SOLUTIONS COMPRISING 3,4 DIHYDROXYPHENYL GROUPS
DISPERSIONS OU SOLUTIONS POLYMERES COMPORTANT DES GROUPES 3,4-DIHYDROXYPHENYLE

(30) Priorität: 27.01.2004 DE 102004004214
(43) Veröffentlichungstag der Anmeldung: 18.10.2006
(73) Patentinhaber: BASF SE, 67056 Ludwigshafen (DE)
(72) Erfinder: URBAN, Dieter, 67346 Speyer (DE); SCHWALM, Reinhold, 67157 Wachenheim (DE); KIRSCH, Stefan, 55268 Nieder-Olm (DE); EXNER, Kai, Michael, 69214 Eppelheim (DE); CENTNER, Alexander, 67127 Rödersheim-Gronau (DE)
(86) Internationale Anmeldenummer: PCT/EP2005/000422
(87) Internationale Veröffentlichungsnummer: WO 2005/070866

(56) Entgegenhaltungen:
- EP-A- 0 003 516
- US-A- 5 326 826
- US-A- 5 623 014
- DATABASE HCAPLUS [Online] 9. Februar 1998 (1998-02-09), AOSHIMA, KATSATARO: "Negative-working lithographic printing plate with improved printing durability" XP002324986 gefunden im STN Database accession no. 1998:76151 & PATENT ABSTRACTS OF JAPAN Bd. 1998, Nr. 06, 30. April 1998 (1998-04-30) & JP 10 029292 A (FUJI PHOTO FILM), 3. Februar 1998 (1998-02-03)
- DATABASE REGISTRY [Online] 26. März 1998 (1998-03-26), XP002324987 gefunden im STN

## Beschreibung

Die Erfindung betrifft die Verwendung einer Dispersion oder Lösung eines unten näher bezeichneten Polymeren in Wasser, organischen Lösemitteln oder deren Gemischen als Klebstoffe, insbesondere Haftklebstoffe, Dichtungsmasse, Imprägnierungsmittel oder Beschichtungsmittel. **dadurch gekennzeichnet, dass** das Polymer mindestens 0,001 Mol 3,4 Dihydroxyphenylgruppen (berechnet mit 109 g/Mol) auf 100 g Polymer enthält.

Bei Polymeren, welche in Beschichtungsmitteln oder Klebstoffen Verwendung finden, handelt es sich vielfach um vernetzungsfähige Copolymerisate. Durch eine Vernetzung können z.B. Schutzüberzüge oder Klebstoffbeschichtungen mit guten elastischen Eigenschaften, hoher Kohäsion, d.h. innere Festigkeit, hoher Chemikalien- und Lösemittelbeständigkeit erhalten werden.

Zur Vernetzung wird den Copolymerisaten im allgemeinen ein Vernetzungsmittel zugesetzt, das mit funktionellen Gruppen im Copolymerisat reagiert.

Mögliche Vernetzungsmittel sind z.B. Polyisocyanate, welche mit Hydroxyl- oder Aminogruppen reagieren.

Nachteilig bei diesen wässrigen Zubereitungen ist jedoch die mangelnde Lagerstabilität. Das Polyisocyanat darf daher erst kurz vor seiner Verwendung als Vemetzungshilfsmittel in Wasser dispergiert und mit dem Copolymerisat gemischt werden.

Eine erhöhte Lagerstabilität kann durch Umsetzung der Isocyanatgruppen mit Blockierungsmitteln, z.B. Oximen, Caprolactam, Phenolen, Maleinsäuredialkylestern erreicht werden. Die erhaltenen sog. blockierten Polyisocyanate hydrolysieren in wässriger Dispersion nur noch in untergeordnetem Ausmaß.

Vernetzungsreaktionen treten jedoch erst nach Abspaltung des Blockierungsmittels bei Temperaturen ab ca. 130°C auf.

Bisher bekannte wässrige Klebstoffzubereitungen mit Polyisocyanaten als Vernetzungshilfsmittel sind daher entweder nicht lagerstabil und können daher nur als 2-Komponentensystem Verwendung finden oder vernetzen erst bei hohen Temperaturen.

Lagerstabile, bei Raumtemperatur nach Entfernen des Lösungsmittels vernetzende wässrige Dispersionen sind z.B. aus der EP-A-3516, WO 93/25588 oder EP-A-516074 bekannt. Diese Dispersionen enthalten Polyhydrazide bzw. Aminooxy-Vernetzer, welche mit im Copolymerisat einpolymerisierten Monomeren mit Carbonylgruppen reagieren.

Grundsätzlich besteht ein Bedarf an weiteren, bei Raumtemperatur vernetzenden Dispersionen, um Alternativen zur Polyhydrazidvernetzung zur Verfügung stellen zu können. Des weiteren sollen diese Dispersionen gute anwendungstechnische Eigenschaften aufweisen.

Aus der Natur sind Proteine mit 3,4 Dihydroxyphenylgruppen bekannt. Diese Proteine bewirken bei Muscheln eine pH-Wert abhängige Vernetzung in Gegenwart von Sauerstoff.

Aufgabe der vorliegenden Erfindung waren daher lagerstabile Dispersionen oder Lösungen von vernetzbaren Polymeren. Die Vernetzung soll durch eine einfache Maßnahme gezielt eingeleitet werden können.

Demgemäß wurde die obige Dispersion oder Lösung gefunden.

Die erfindungsgemäße Dispersion oder Lösung enthält ein Polymer mit mindestens 0,001 Mol 3,4 Dihydroxyphenylgruppen auf 100 g Polymer.

Die Mindestmenge der 3,4 Dihydroxyphenylgruppen beträgt vorzugsweise 0,001, besonders bevorzugt 0,005 bzw. 0,02 Mol auf 100 g Polymer; die Maximalmenge übersteigt vorzugsweise nicht 0,5, besonders bevorzugt nicht 0,2 Mol auf 100 g Polymer.

Eine ausreichende Vernetzung wird im allgemeinen mit einem Gehalt von 0,001 bis 0,2, insbesondere 0,01 bis 0,15 und besonders bevorzugt von 0,05 bis 0,1 erreicht.

Unter 3,4 Dihydroxyphenylgruppe wird dabei eine Gruppe der Formel 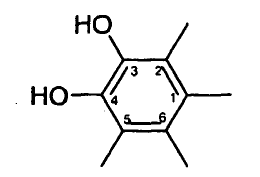 verstanden.

An der 1., 2., 5. und 6. Position befinden sich gegebenenfalls weitere Substituenten, in Betracht kommen insbesondere H-Atome oder Kohlenwasserstoffgruppen ggfs. auch mit Heteroatomen.

Zur Berechnung des molaren Gehalts im Polymer wird immer von einem Molgewicht der 3,4 Dihydroxygruppe von 109 Gramm pro Mol ausgegangen.

Die erfindungsgemäße Dispersion oder Lösung kann als Lösungsmittel Wasser oder bei 21 °C, 1 bar flüssige organische Lösungsmittel enthalten.

Bevorzugt ist Wasser oder Gemische von Wasser und mit Wasser mischbare organische Lösemittel.

Bei Lösemittelgemischen beträgt der Anteil von Wasser vorzugsweise mindestens 50 Gew.%, bezogen auf das Lösemittelgemisch.

Es handelt sich um ein Polymer, welches durch radikalische Polymerisation von ethylenisch ungesättigten Verbindungen (Monomere) erhältlich ist (im nachfolgenden kurz Polyaddukt genannt).

Das Polyaddukt besteht zu mindestens 40 Gew.-%, bevorzugt zu mindestens 60 Gew.-%, besonders bevorzugt zu mindestens 80 Gew.-% aus sogenannten Hauptmonomeren.

Die Hauptmonomeren sind ausgewählt aus C₁-C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C-Atome enthaltenden Alkoholen, aliphatischen Kohlenwasserstoffen mit 2 bis 8 C-Atomen und ein oder zwei Doppelbindungen oder Mischungen dieser Monomeren.

Zu nennen sind z.B. (Meth)acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, -stearat, Vinylpropionat, Versaticsäurevinylester und Vinylacetat.

Als vinylaromatische Verbindungen kommen Vinyltoluol, α- und p-Methylstyrol, α-Butylstyrol, 4-n-Butylstyrol, 4-n-Decylstyrol und vorzugsweise Styrol in Betracht. Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als Vinylether zu nennen sind z.B. Vinylmethylether oder Vinylisobutylether. Bevorzugt wird Vinylether von 1 bis 4 C-Atome enthaltenden Alkoholen.

Als Kohlenwasserstoffe mit 2 bis 8 C-Atomen und ein oder zwei olefinischen Doppelbindungen seien Ethylen, Propylen, Butadien, Isopren und Chloropren genannt.

Bevorzugte Hauptmonomere sind C₁-C₁₀-Alkyl(meth)acrylate und Mischungen der Alkyl(meth)acrylate mit Vinylaromaten, insbesondere Styrol.

Neben den Hauptmonomeren kann das Polymer weitere Monomere enthalten, z.B. Monomere mit Carbonsäure, Sulfonsäure oder Phosphonsäuregruppen. Bevorzugt sind Carbonsäuregruppen. Genannt seien z.B. Acrylsäure, Methacrylsäure, Itaconsäure, Maleinsäure oder Fumarsäure. Der Gehalt an ethylenisch ungesättigten Säuren im Polymerisat ist im allgemeinen kleiner 15 Gew.-%.

Weitere Monomere sind z.B. auch Hydroxylgruppen enthaltende Monomere, insbesondere C₁-C₁₀-Hydroxyalkyl(meth)acrylate, oder Amide wie (Meth)acrylamid.

Im Polyaddukt sind die 3,4 Dihydroxyphenylgruppen durch Copolymerisation mit 3,4 Dihydroxyphenylgruppen aufweisenden Monomeren enthalten.

Als geeignete 3,4 Dihydroxyphenylgruppen aufweisende Monomere kommen solche der Formel 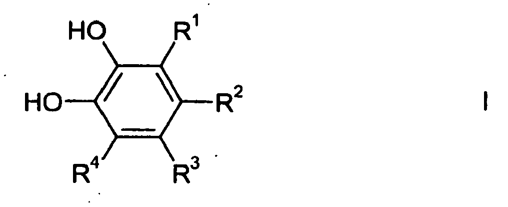 in Betracht.

Mindestens einer der Reste R1 bis R⁴ steht hier für einen organischen Rest der eine radikalisch polymerisierbare, ethylenisch ungesättigte Gruppe enthält (kurz ethylenisch ungesättigter Rest).

Der ethylenisch ungesättigte Rest enthält im übrigen bevorzugt insgesamt bis zu 50 C-Atome, insbesondere bis zu 30 C-Atome und gegebenenfalls auch Heteroatome wie O, N und S. Derartige Heteroatome können insbesondere in Form von Carbonyl-, Carboxyl-, Hydroxy- Ether-, Amino- oder Mercaptogruppen vorliegen.

Besonders bevorzugt steht einer der Reste R¹ bis R⁴ für einen ethylenisch ungesättigten Rest.

Ganz besonders bevorzugt steht R² für einen ethylenisch ungesättigten Rest.

Die übrigen Reste stehen für Wasserstoff oder sonstige organische Reste ohne radikalisch copolymerisierbare Gruppe. Die übrigen Reste stehen bevorzugt für Wasserstoff oder C₁-C₈-Alkylgruppen. Besonders bevorzugt handelt es sich bei maximal zwei der übrigen Reste um organische Reste, insbesondere handelt es sich bei mindestens einem, vorzugsweise bei allen übrigen Resten um Wasserstoff.

Mindestens einer der Reste R¹ bis R⁴ ist ein Rest der Formel -Y-X, wobei Y eine Spacergruppe und X die eigentllich copolymerisierbare Gruppe ist. X ist ausgewählt aus:

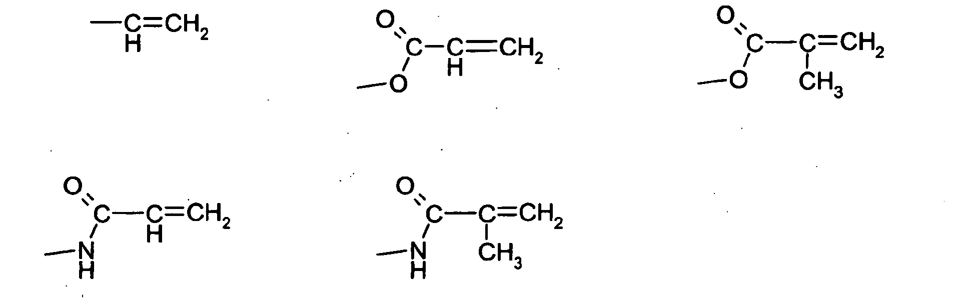

Die Spacergruppe Y steht im einfachsten Fall für eine Einfachbindung, die Gruppe X ist dann direkt an den Phenylring gebunden.

Im übrigen kann es sich bei Y um eine zweiwertige Spacergruppe mit bis zu 30 C-Atomen, insbesondere bis zu 20 C-Atomen, ganz besonders bevorzugt bis zu 15 C-Atomen handeln.

Die Spacergruppe kann auch Heteroatome wie O, N und S enthalten, z.B. in Form von Carbonyl-, Carboxyl-, Hydroxy-, Ethan-, Amino- oder Mercaptogruppen.

Besonders bevorzugte Monomere mit 3,4 Dihydroxyphenylgruppen sind radikalisch polymerisierbare Monomere mit 3,4 Dihydroxyphenylgruppen und mindestens einer radikalische polymerisierbaren Doppelbindung, welche erhältlich sind durch Umsetzung von Verbindungen I mit einer 3,4 Dihydroxyphenylgruppe, welche durch mindestens einen weiteren organischen Rest mit einer Hydroxylgruppe oder Carboxylgruppe substituiert ist, mit Verbindungen II, welche mindestens eine radikalisch polymerisierbare Doppelbindung und mindestens eine gegenüber Verbindungen I reaktive Gruppe, z.B. ein Hydroxy-, Carboxyl- oder Epoxygruppe aufweisen.

Ganz besonders bevorzugt sind Monomere aus Verbindungen I mit einer Hydroxyalkylgruppe und ethylenisch ungesättigten Säuren als Verbindungen II. Als Verbindungen I mit Hydroxyalkylgruppen kommen insbesondere solche in Betracht, bei denen ein 3,4-Dihydroxyphenyl durch eine C₂-C₁₀-Alkanolgruppe, z.B. Ethanol substituiert ist.

Als ethylenisch ungesättigte Säuren kommen insbesondere Acrylsäure, Methacrylsäure, Itaconsäure in Betracht.

Bei der Umsetzung von I mit II handelt es sich um eine normale Veresterung. Die Umsetzung kann bei 40 bis 100°C in Gegenwart von Säuren, z.B. Schwefelsäure erfolgen.

Ganz besonders bevorzugt sind ebenfalls Monomere aus Verbindungen I mit einer Carboxylgruppe und ethylenisch ungesättigten Epoxiden als Verbindungen II.

Hier kommen als Verbindungen I insbesondere solche in Betracht, bei denen ein 3,4-Dihydroxyphenol durch eine C₁-C₁₀-Alkylcarboxylgruppe substituiert ist.

Als Verbindungen II kommen insbesondere Glycidylacrylat und Glycidylmethacrylat in Betracht. Die Umsetzung erfolgt vorzugsweise bei 40 bis 100°C in Gegenwart eines Katalysators für die Öffnung des Epoxidrings.

Als radikalisch polymerisierbare Monomere mit 3,4 Dihydroxyphenylgruppen und mindestens einer radikalisch polymerisierbaren Doppelbindung kommen auch Säureamide in Betracht, bei denen die Amidgruppen einen Substituenten mit einer 3,4 Dihydroxylphenylgruppe hat.

Insbesondere handelt es sich um entsprechend substituierte Acrylsäure- oder Methacrylsäureamide.

Die Säureamide sind z.B. erhältlich durch Umsetzung von Brenzkatechin mit (Meth)acrylsäureamiden, bei denen die Amidgruppe eine Hydroxylgruppe trägt.

N-[2-(3,4-dihydroxyphenyl)ethyl]-2-propenamid ist z.B. aus Chemical abstracts Nr. S 203179-84-4 bekannt.

Das Polyaddukt kann in üblicher Weise durch Copolymerisation der Monomeren hergestellt werden.

Der Anteil der 3,4 Dihydroxyphenylgruppen enthaltenden Monomeren sind so gewählt, dass der gewünschte Gehalt an 3,4 Dihydroxyphenylgruppen im Polyaddukt erreicht wird.

Das Polyaddukt kann ausschließlich aus 3,4 Dihydroxyphenylgruppen enthaltenden Monomeren aufgebaut sein.

Insbesondere enthält die erfindungsgemäße Lösung oder Dispersion ein Polymer, vorzugsweise ein Polyaddukt, mit 0,001 bis 0,7 Mol 3,4 Dihydroxyphenylgruppen auf 100 g Polymer. Besonders bevorzugt sind die eingangs angegebenen Mindest- und Maximalmengen.

Die Polymeren sind z.B. durch Lösungspolymerisation oder Emulsionspolymerisation erhältlich.

Bevorzugt werden dabei Wasser oder wässrige Lösungsmittelgemische mit insbesondere mehr als 50 Gew.-% Wasseranteil als Lösungsmittel verwendet.

Ein besonderer Vorteil der Vernetzungsreaktion der 3,4 Dihydroxyphenylgruppen ist die pH-Wert-Abhängigkeit.

Bei einem pH-Wert kleiner 4 tritt keine Vernetzung auf.

Ebenso tritt bei Sauerstoffausschluß keine Vernetzung auf. Zur Vernetzung muß der pH-Wert größer 4, besonders bevorzugt größer 6, ganz besonders bevorzugt größer 7 sein; gleichzeitig muß Gegenwart von Sauerstoff gegeben sein.

Die unvernetzte Lösung oder Dispersion kann entsprechend bei einem beliebigen pH-Wert unter Sauerstoffausschluß gelagert werden. Alternativ kann die unvemetzte Lösung oder Dispersion in Gegenwart von Sauerstoff bei einem pH-Wert kleiner 7, insbesondere kleiner 4 gelagert werden.

Bei der Verwendung der Lösung oder Dispersion erfolgt dann die Vernetzung des gelösten oder dispergierten Polymeren durch Anhebung des pH-Wertes oder durch Aufhebung des Sauerstoffausschusses oder durch beides.

Die Verwendung kann auch unter Wasser erfolgen, in Gegenwart des in Wasser gelösten Sauerstoffs.

Die erfindungsgemäße Dispersion oder Lösung kann je nach Verwendungszweck übliche Hilfs- und Zusatzmittel enthalten. Hierzu gehören beispielsweise Füllstoffe wie Quarzmehl, Quarzsand, hochdisperse Kieselsäure, Schwerspat, Calciumcarbonat, Kreide, Dolomit oder Talkum, die oft zusammen mit geeigneten Netzmitteln wie z.B. Polyphosphaten wie Natriumhexamethaphosphat, Naphthalinsulfonsäure, Ammonium- oder Natriumpolyacrylsäuresalze eingesetzt werden, wobei die Netzmittel im allgemeinen von 0,2 bis 0,6 Gew.-%, bezogen auf den Füllstoff, zugesetzt werden.

Fungizide zur Konservierung werden, falls gewünscht im allgemeinen in Mengen von 0,02 bis 1 Gew.-%, bezogen auf die gesamte Dispersionen oder Lösung eingesetzt. Geeignete Fungizide sind beispielsweise Phenol- oder Kresol-Derivate oder zinnorganische Verbindungen.

Besonders eignet sich die erfindungsgemäße Dispersion oder Lösung, insbesondere als wässrige Dispersion eines radikalischen Polymeren, als Bindemittel für Klebstoffe, z.B. Haftklebstoffe, Lacke, Anstriche, Papierstreichmassen oder zum Binden von Faservliesen, d.h. überall wo eine Vernetzung und Erhöhung der inneren Festigkeit (Kohäsion) gewünscht ist. Als Klebstoff können die Dispersionen neben obengenannten Zusatzstoffen noch spezielle, in der Klebstofftechnologie übliche Hilfs- und Zusatzmittel enthalten. Hierzu gehören beispielsweise Verdickungsmittel, Weichmacher oder auch klebrigmachende Harze wie z.B. Naturharze oder modifizierte Harze wie Kolophoniumester oder synthetische Harze wie Phthalatharze.

Dispersionen, welche als Klebstoff Verwendung finden, enthalten besonders bevorzugt Alkyl(meth)acrylate im Copolymerisat. Bevorzugte Anwendungen im Klebstoffgebiet sind neben Haftklebstoffen auch Kaschierklebstoffe, z.B. für die Verbund- und Glanzfolienkaschierung.

Die Glasübergangstemperatur der Polymeren wird bei der Verwendung als Klebstoff bevorzugt auf Werte zwischen 0 und -60°C eingestellt.

### Beispiele

### Dihydroxylphenylverbindungen:

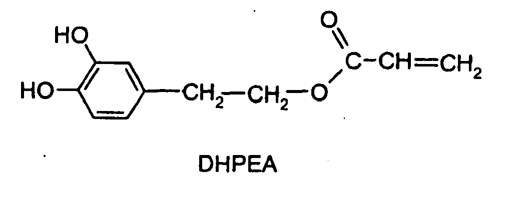

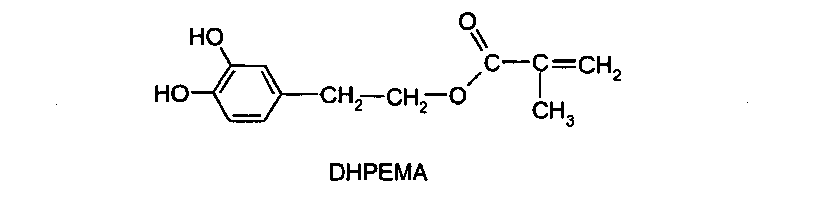

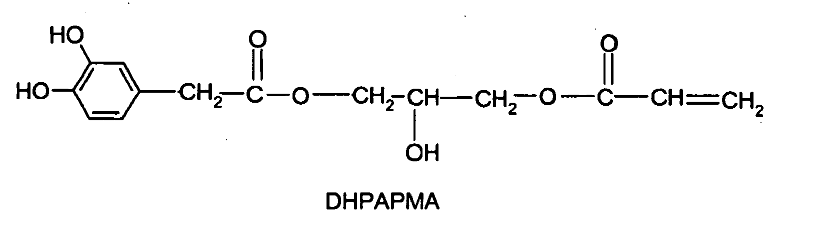

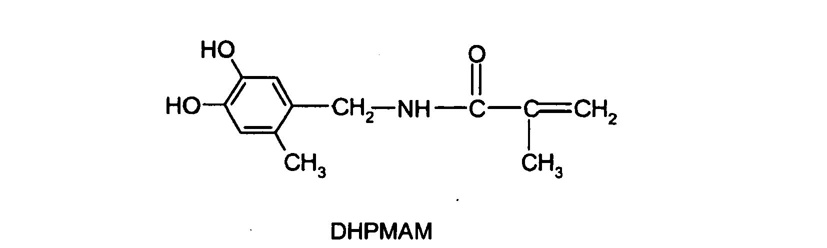

### Beispiel A) Herstellung von DHPEA

100 Teile 2-(3,4 dihydroxyphenyl) ethanol, 100 Teile Acrylsäure, 4 Teile Schwefelsäure, 0,1 Teile Phenothiazin, 0,5 Teile Hydrochinon monomethylester und 50 Teile Cyclohexan werden am Wasserauskreiser so lange erhitzt, bis 9 Teile Wasser ausgekreist sind.

Nach dem Abkühlen gibt man 350 Teile Ethylacetat zum Reaktionsgemisch, schüttelt dreimal mit Kochsalzlösung aus, trocknet die organische Phase mit Natriumsulfat und rotiert das Lösungsmittel ab. Das so erhaltene Gemisch aus 2-(3,4 dihydroxyphenyl) ethyl acrylat (DHPEA) und Acrylsäure (charakterisiert über 1H-NMR) in einer Ausbeute von 110 Teilen wird so in der anschließenden Emulsionspolymerisation eingesetzt.

### Beispiel B) Herstellung von DHPEA

100 Teile 2-(3,4 dihydroxyphenyl) ethanol, 100 Teile Acrylsäure, 4 Teile p-Toluolsulfonsäure, 0,1 Teile Phenothiazin, 0,5 Teile Hydrochinon monomethylester und 50 Teile Cyclohexan werden am Wasserauskreiser so lange erhitzt, bis 9 Teile Wasser ausgekreist sind.

Nach dem Abkühlen gibt man 350 Teile Ethylacetat zum Reaktionsgemisch, schüttelt dreimal mit Kochsalzlösung aus, trocknet die organische Phase mit Natriumsulfat und rotiert das Lösungsmittel ab. Das so erhaltene Gemisch aus 2-(3,4 dihydroxyphenyl) ethyl acrylat und Acrylsäure (charakterisiert über 1H-NMR) in einer Ausbeute von 88 Teilen wird so in der anschließenden Emulsionspolymerisation eingesetzt.

### Beispiel C) Herstellung von DHPEMA

55 Teile 2-(3,4 dihydroxyphenyl) ethanol, 56 Teile Methacrylsäureanhydrid, 0,1 Teile Phenothiazin, 0,5 Teile Hydrochinon monomethylester und 50 Teile Tetrahydrofuran werden bei Raumtemperatur zusammengegeben und 1 Stunde gerührt, dann wird die Temperatur auf 70°C erhöht, 1 Teil Dibutylzinndilaurat und 4 Teile Triethanolamin zugegeben und weitere 3 Stunden bei 70°C reagieren lassen.

Nach dem Abkühlen gibt man 100 Teile Ethylacetat zum Reaktionsgemisch, säuert mit Salzsäure auf pH5 und schüttelt 2 mal mit Kochsalzlösung aus und trocknet anschließend die organische Phase mit Natriumsulfat und rotiert das Lösungsmittel ab. Das so erhaltene Gemisch aus 2-(3,4 dihylroxyphenyl) ethyl methacrylat und Methacrylsäure (charakterisiert über 1H-NMR) in einer Ausbeute von 90 Teilen wird so in der anschließenden Emulsionspolymerisation eingesetzt.

### Beispiel D) Synthese über Schutzgruppe für phenolische OH

100 Teile 2-(3,4 dihydroxyphenyl) ethanol und 120 Teile Aceton werden bei 0°C vorgelegt und 180 Teile Phosphorpentoxid werden langsam zugegeben. Es resultiert eine exotherme Reaktion auf ca. 30°C, danach wird weitere 4 Stunden bei Raumtemperatur weiterreagieren lassen. Es wird Ethylacetat zugesetzt, das Produkt mit Natronlauge gewaschen, die organische Phase mit Natriumchlorid ausgeschüttelt, mit Natriumsulfat getrocknet und einrotiert. Es resultieren 50 Teile gelbliche Kristalle. Diese können dann in klassischen Reaktionen mit Acrylsäurechlorid oder Methacrylsäureanhydrid umgestezt und anschließend die Schutzgruppe wieder abgespalten werden.

### Beispiel E) Herstellung von DHPAPMA

20 Teile 1-(3,4 dihydroxyphenyl) essigsäure, 17 Teile Glycidylmethacrylat, 20 Teile Butylacetat und 0,7 Teile Tetrabutylammoniumbromid, 0,1 Teile Phenothiazin und 0,5 Teile Hydrochinon monomethylester werden bei Raumtemperatur zusammengegeben und dann 10 Stunden bei 80°C gerührt. Es resultiert eine schwache exotherem Reaktion. Das Produkt wird über H-NMR Spektroskopie identifiziert. Das so erhaltene Produkt wird so in der anschließenden Emulsionspolymerisation eingesetzt.

### Herstellung der Polymeren

### Lösungspolymerisation:

Polymer mit 5% DHPEA

In einen 100 ml Rundkolben mit Magnetrührer und geeignetem Verschluss mit N2-Zufuhr und Abgasleitung gibt man eine Mischung aus 80g Aceton, 15,2g 2-Hydroxyethylacrylat (HEA) und 0,8g 3,4-Dihydroxyphenylethylacrylat (DHPEA), zusammen mit einer Mischung aus 80g Wasser, 0,08g Natriumdisulfit, 0,16g 2,2' Azobis-(2-amidinopropan)-dihydrochlorid (Wako V50).

Die Mischung wird bei Raumtemperatur 50 min mit Stickstoff gespült und gerührt. Bei konstantem Stickstoffstrom erwärmt man die Mischung auf 60°C, rührt eine Stunde bei dieser Temperatur, destilliert das Aceton ab und hält die Reaktionsmischung eine weitere Stunde bei 80°C.

Die erhaltene Polymerlösung ist farblos und hat einen pH-Wert von 2,7 und einen Feststoffgehalt von ca. 20%.

Analog wurden weitere Lösungspolymerisate mit unterschiedlichen Mengen DHPEA bzw. 10% (3,4-Dihydroxy-6-methyl-phenyl)-methyl-(meth)acrylamid (DHPMAM) hergestellt. Die Zusammensetzungen und Filmeigenschaften sind in Tabelle 1 aufgeführt.

**Tabelle 1:**

| Beispiel | HEA (%) | DHPEA (%) | FG(%) | pH | Film | Farbe |
|---|---|---|---|---|---|---|
| 1 | 100 | | ca. 20 | 3,8 | klebrig | klar |
| 2a | 99 | 1 | ca. 20 | 2,7 | klebrig | klar |
| 2b | 99 | 1 | ca. 20 | 8,5 | klebrig | hellbraun |
| 3a | 95 | 5 | ca. 20 | 2,5 | klebrig | klar |
| 3b | 95 | 5 | ca. 20 | 8,5 | nicht klebrig | braun |
| 4a | 80 | 10 | ca. 20 | 2,2 | klebrig | hellbraun |
| 4b | 80 | 10 | ca. 20 | 8,5 | nicht klebrig | dunkelbraun |
| | HEA (%) | DHPMAM (%) | FG(%) | pH | Film | Farbe |
| 5a | 90 | 10 | ca. 20 | 2,5 | klebrig | klar |
| 5b | 90 | 10 | ca. 20 | 8,0 | nicht klebrig | dunkelbraun |

Von den Polymerlösungen wurden ca. 15g in einen Polyethylendeckel (Masse 7g) gegeben und bei Raumtemperatur getrocknet (Beispiele 1, 2a bis 5a). Ein Teil der Polymerlösungen wurde mit 5%iger NaOH auf pH 8-8,5 gestellt. Von den alkalisch gestellten Polymerlösungen wurden auch ca. 15g in einen Polyethylendeckel gegeben und unter den gleichen Bedingungen getrocknet (Beispiele 2b bis 5b).

Die aus saurer Lösung gebildeten Filme waren alle klebrig und größtenteils klar. Die aus alkalischer Lösung gebildeten Filme zeigten eine Braunfärbung, die mit steigender Menge DHPEA zunahm. Gleichzeitig nahm mit steigender Braunfärbung die Klebrigkeit ab. Die Abnahme der Klebrigkeit ist ein Maß für die Zunahme der Vernetzungsdichte.

Definitionen: Der Film wird als klebrig bezeichnet, wenn der Polyethylendeckel sich mit einem Finger hochheben lässt, nachdem die Oberfläche des Polymerfilms mit dem Finger unter leichtem Anpressdruck kurz berührt worden war. Nicht klebrig bedeutet, dass der Polyethylendeckel liegen bleibt.

### Emulsionspolymerisation:

Polymer mit 5% DHPEA

In einen 100 ml Rundkolben mit Magnetrührer und geeignetem Verschluss mit N2-Zufuhr und Abgasleitung gibt man eine Mischung aus 7,6g n-Butylacrylat (BA) und 0,4g 3,4-Dihydroxyphenylethylacrylat (DHPEA), zusammen mit einer Mischung aus 72,9g Wasser, 0,267g Steinapol NLS (15%ig in Wasser), 0,08g 2,2'-Azobis[N-(2-carboxyethyl)-2-methylpropionamidin] tetrahydrat (VA 057).

Die Mischung wird bei Raumtemperatur 30 min mit Stickstoff gespült und gerührt. Bei konstantem Stickstoffstrom erwärmt man die Mischung auf 85°C, rührt drei Stunden bei dieser Temperatur und kühlt ab. Die erhaltene Polymerdispersion ist weiß und hat einen pH-Wert von 2,8 und einen Feststoffgehalt von 10% (Beispiel 8a).

Analog wurden weitere Emulsionspolymerisate mit unterschiedlichen Mengen DHPEA hergestellt. Die Zusammensetzungen sind in Tabelle 2 aufgeführt.

**Tabelle 2**

| Beispiel | BA (%) | DHPEA (%) | FG(%) | pH | Farbe des Films | Tack (J/mm2) | Tack(pH9)/Tack(pH3) |
|---|---|---|---|---|---|---|---|
| 6a | 100 | | ca.10 | 2,5 | farblos | 72 ± 24 | |
| 6b | 100 | | ca.10 | 9,0 | farblos | 63 ± 9 | 0,88 |
| 7a | 99 | 1 | ca.10 | 3,3 | farblos | 205 ± 67 | |
| 7b | 99 | 1 | ca.10 | 9,0 | fast farblos | 88 ± 10 | 0,43 |
| 8a | 95 | 5 | ca.10 | 2,8 | farblos | 115 ± 18 | |
| 8b | 95 | 5 | ca.10 | 9,0 | hellbraun | 31 ± 6 | 0,27 |
| 9a | 80 | 10 | ca.10 | 2,6 | hellbraun | 126 ± 23 | |
| 9b | 80 | 10 | ca.10 | 9,0 | dunkelbraun | 10 ± 2 | 0,08 |

Für die Tack-Messungen wurden die Polymerdispersionen mit einem 400µm Rakel auf eine Glasplatte aufgetragen und die Filme über Nacht bei Raumtemperatur getrocknet (Beispiele 6a bis 9a). Ein Teil der Polymerdispersionen wurde mit 2%iger NaOH auf pH 9,5 gestellt. Von den alkalisch gestellten Polymerdispersionen wurden 15 Minuten nach pH Stellung auch 400µm Filme auf eine Glasplatte gerakelt und ebenso über Nacht bei Raumtemperatur getrocknet (Beispiele 6b bis 9b).

Die Messung erfolgte nach 24-stündiger Trocknungszeit mit dem TA.XT.plus Tackmessgerät. (Stahlstempel d=2mm, Anpresskraft 1N, Kontaktzeit 1s, Abzugsgeschwindigkeit 1mm/s, Filmdicke 450µm nass, Temperatur 24°C)

Tack ist die Fläche unter der Kraft-Zeit-Kurve, die beim Abziehen des Stahlstempels entsteht.

Als Referenz wurde eine Butylacrylatdispersion verwendet, deren im Sauren (6a) und alkalischen (6b) hergestellten Filme keine signifikante Veränderung des Tack zeigen (die Abnahme von 72 auf 63 J/mm2 liegt im Bereich des Messfehlers).

Deutlich größer ist der Tackunterschied bei den Proben, die DHPEA enthalten. Bei 1 % DHPEA sinkt der Tack auf 43% des Ausgangswertes, bei 5% DHPEA auf 27% und bei 10% DHPEA sogar auf 8% des Ausgangswertes ab.

Die Tackabnahme wird durch die Vernetzungsreaktion der Dihydroxyphenyleinheiten verursacht, die bei Raumtemperatur in Gegenwart von Sauerstoff im alkalischen eintritt. Schwermetallionen können diese Reaktion noch verstärken.

## Patentansprüche

1. Verwendung einer Dispersion oder Lösung eines Polymeren in Wasser, organischen Lösemitteln oder deren Gemischen, als Klebstoff, Dichtungsmasse, Beschichtungsmittel oder Imprägnierungsmittel, **dadurch gekennzeichnet, dass** das Polymer mindestens 0,001 Mol 3,4 Dihydroxyphenylgruppen (berechnet mit 109 g/Mol) auf 100 g Polymer enthält, wobei es sich bei dem Polymeren um ein durch radikalische Polymerisation von ethylenisch ungesättigten Verbindungen erhältliches Polymer handelt, wobei das Polymer zu mindestens 40 Gew.-% aus sogenannten Hauptmonomeren, ausgewählt aus C₁- bis C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C-Atome enthaltenden Alkoholen, aliphatischen Kohlenwasserstoffen mit 2 bis 8 C-Atomen und ein oder zwei Doppelbindungen oder Mischungen dieser Monomeren, aufgebaut ist, wobei die 3,4 Dihydroxyphenylgruppen durch Copolymerisation mit 3,4 Dihydroxyphenylgruppen aufweisenden Monomeren im Polymer enthalten sind, wobei es sich bei den 3,4 Dihydroxyphenylgruppen aufweisenden Monomeren um solche der Formel 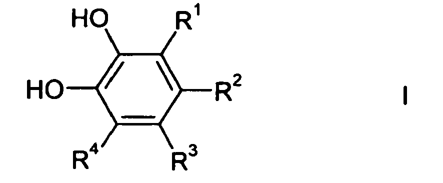 handelt, in der mindestens einer der Reste R¹ bis R⁴ für eine Gruppe -Y-X steht, wobei X ausgewählt ist aus 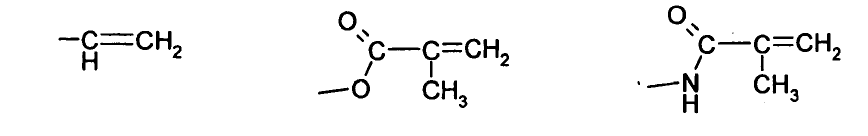 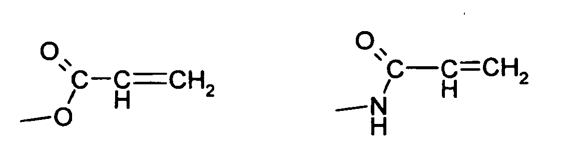 und Y für eine Einfachbindung oder für eine zweiwertige Spacergruppe mit bis zu 30 C-Atomen und gegebenenfalls Heteroatomen wie O, N oder S steht und die übrigen Reste für organische Reste ohne copolymerisierbare Gruppe oder für Wasserstoff stehen.

2. Verwendung gemäß Anspruch 1, **dadurch gekennzeichnet, dass** es sich bei der Dispersion oder Lösung um eine wässrige Dispersion oder Lösung handelt.

3. Verwendung gemäß einem der Ansprüche 1 bis 2, **dadurch gekennzeichnet, dass** die Glasübergangstemperatur des Polymeren kleiner +10°C, vorzugsweise kleiner 0°C ist.

4. Verwendung gemäß einem der Ansprüche 1 bis 3, **dadurch gekennzeichnet, dass** der pH-Wert der Dispersion oder Lösung kleiner 7 ist.

5. Verwendung gemäß einem der Ansprüche 1 bis 4, **dadurch gekennzeichnet, dass** die Dispersion oder Lösung vor der Verwendung sauerstofffrei gelagert wird und erst bei der Verwendung in Kontakt mit Sauerstoff kommt.

6. Verwendung gemäß einem der Ansprüche 1 bis 5, **dadurch gekennzeichnet, dass** die Dispersion oder Lösung vor der Verwendung einen pH-Wert kleiner 4 hat und dieser pH-Wert bei der Verwendung auf über 4 erhöht wird.

7. Verwendung gemäß einem der Ansprüche 1 bis 6, **dadurch gekennzeichnet, dass** die Verwendung unter Wasser erfolgt.

## Claims

1. The use of a dispersion or solution of a polymer in water, organic solvents or mixtures thereof, as adhesive, sealant, coating material or impregnating composition, wherein the polymer comprises at least 0.001 mol of 3,4 dihydroxyphenyl groups (calculated at 109 g/mol) per 100 g of polymer, the polymer being a polymer obtainable by free-radical addition polymerization of ethylenically unsaturated compounds, the polymer being synthesized from at least 40% by weight of principal monomers selected from C₁ to C₂₀ alkyl (meth) acrylates, vinyl esters of carboxylic acids comprising up to 20 carbon atoms, vinylaromatics having up to 20 carbon atoms, ethylenically unsaturated nitriles, vinyl halides, vinyl ethers of alcohols comprising 1 to 10 carbon atoms, aliphatic hydrocarbons having 2 to 8 carbon atoms and one or two double bonds or mixtures of these monomers, the 3,4 dihydroxyphenyl groups being present in the polymer by copolymerization with monomers containing 3,4 dihydroxyphenyl groups, the monomers containing 3,4 dihydroxyphenyl groups being those of the formula 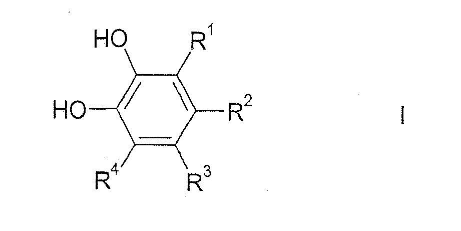 in which at least one of the radicals R¹ to R⁴ is a group -Y-X, where X is selected from 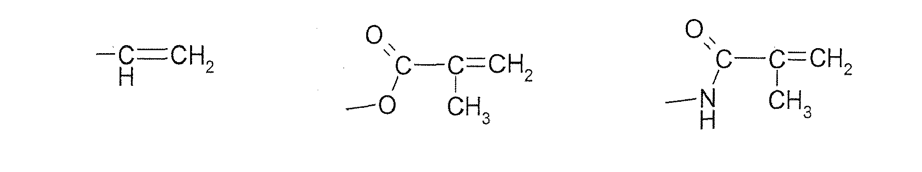 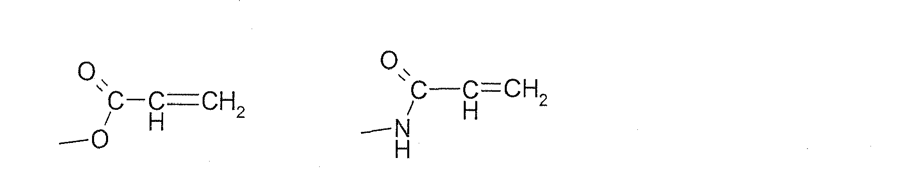 and Y is a single bond or is a divalent spacer group having up to 30 carbon atoms and if appropriate heteroatoms such as O, N or S, and the remaining radicals are organic radicals without a copolymerizable group or are hydrogen.

2. The use according to claim 1, wherein the dispersion or solution is an aqueous dispersion or solution.

3. The use according to one of claims 1 to 2, wherein the glass transition temperature of the polymer is less than +10°C, preferably less than 0°C.

4. The use according to one of claims 1 to 3, wherein the pH of the dispersion or solution is less than 7.

5. The use according to one of claims 1 to 4, wherein the dispersion or solution is stored oxygen-free prior to use and comes into contact with oxygen only upon use.

6. The use according to one of claims 1 to 5, wherein the dispersion or solution has a pH of less than 4 prior to use and this pH is increased to more than 4 upon use.

7. The use according to one of claims 1 to 6, wherein the use takes place under water.

## Revendications

1. Utilisation d'une dispersion ou solution d'un polymère dans l'eau, des solvants organiques ou des mélanges de ceux-ci, en tant qu'adhésif, matière d'étanchéité, produit de revêtement ou agent d'imprégnation, **caractérisée en ce que** le polymère contient au moins 0,001 mole de groupes 3,4-dihydroxyphényle (calculé à 109 g/mole) pour 100 g de polymère, le polymère consistant en un polymère pouvant être obtenu par polymérisation radicalaire de composés à insaturation éthylénique, le polymère étant constitué à raison d'au moins 40 % en poids de monomères dits principaux, choisis parmi des (méth)acrylates d'alkyle en C₁-C₂₀, des esters vinyliques d'acides carboxyliques contenant jusqu'à 20 atomes de carbone, des composés vinylaromatiques ayant jusqu'à 20 atomes de carbone, des nitriles à insaturation éthylénique, des halogénures de vinyle, des éthers vinyliques d'alcools contenant de 1 à 10 atomes de carbone, des hydrocarbures aliphatiques ayant de 2 à 8 atomes de carbone et comportant une ou deux doubles liaisons, ou de mélanges de ces monomères, les groupes 3,4-dihydroxyphényle étant contenus dans le polymère par copolymérisation avec des monomères comportant des groupes 3,4-dihydroxyphényle, les monomères comportant des groupes 3,4-dihydroxyphényle consistant en ceux de formule 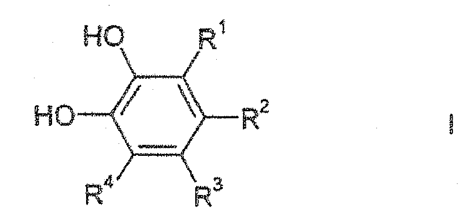 dans laquelle au moins l'un des radicaux R¹ à R⁴ représente un groupe -Y-X, X étant choisi parmi 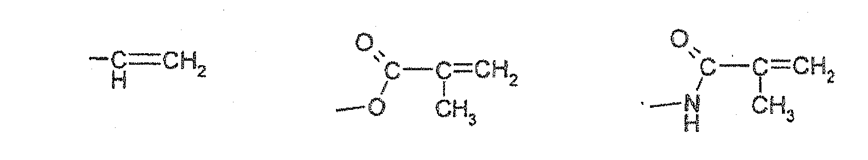 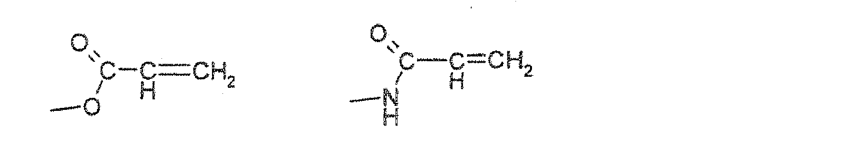 et Y représentant une liaison simple ou un groupe espaceur divalent ayant jusqu'à 30 atomes de carbone et comportant éventuellement des hétéroatomes tels que O, N ou S et les autres radicaux représentant un atome d'hydrogène ou des radicaux organiques sans groupe copolymérisable.

2. Utilisation selon la revendication 1, **caractérisée en ce que** la dispersion ou solution consiste en une dispersion ou solution aqueuse.

3. Utilisation selon l'une quelconque des revendications 1 et 2, **caractérisée en ce que** la température de transition vitreuse du polymère est inférieure à +10 °C, de préférence inférieure à 0 °C.

4. Utilisation selon l'une quelconque des revendications 1 à 3, **caractérisée en ce que** le pH de la dispersion ou solution est inférieur à 7.

5. Utilisation selon l'une quelconque des revendications 1 à 4, **caractérisée en ce que** la dispersion ou solution est stockée à l'abri de l'oxygène avant l'emploi et n'entre en contact avec l'oxygène que lors de l'emploi.

6. Utilisation selon l'une quelconque des revendications 1 à 5, **caractérisée en ce que** la dispersion ou solution a avant l'emploi un pH inférieur à 4 et ce pH est élevé à plus de 4 lors de l'emploi.

7. Utilisation selon l'une quelconque des revendications 1 à 6, **caractérisée en ce que** l'emploi s'effectue sous l'eau.
